# EUROPEAN PATENT APPLICATION

(11) **EP 0 537 556 A1**
(43) Date of publication of application: **21.04.1993**
(21) Application number: 92116758.1
(22) Date of filing: 30.09.1992
(51) Int. Cl.: C07D 209/52, A61K 31/47, C07D 209/44, C07D 401/04

(54) **Unsaturated azabicycloamine compounds and processes for the preparation thereof**

(71) Applicant: KOREA RESEARCH INSTITUTE OF CHEMICAL TECHNOLOGY, Daejeon, 305-606 (KR)
(72) Inventor: Kim, Wan Joo, Yuseng-ku, Daejeon 305-606 (KR); Park, Myung Hwan, Yuseong-ku, Daejeon 305-343 (KR); Ha, Jae Du, Yuseong-ku, Daejeon 305-606 (KR); Baik, Kyong Up, Seo-ku, Daejeon 302-181 (KR)
(74) Representative: Fischer, Hans-Jürgen, Dr.

(57) **Abstract**

The present invention relates to an azabicycloamine compound of the formula (I) and the salts thereof: 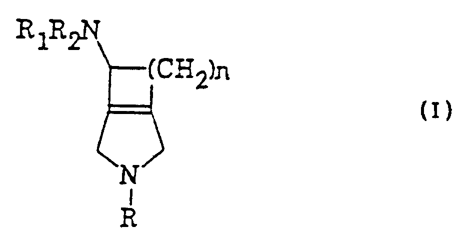
wherein:
R is a hydrogen or an amine protecting group;
R₁ and R₂, which may be the same or different, are hydrogen, optionally substituted lower alkyl, alkanoyl, optionally substituted benzoyl or naphthoyl, optionally substituted alkoxycarbonyl, or optionally substituted benzyloxycarbonyl; and
n is an integer from 1 to 4.

## Description

### Field of the Invention

The present invention relates to unsaturated azabicycloamine compounds and processes for the preparation thereof, and, more specifically, to unsaturated azabicycloamine compounds useful as an intermediate for preparing quinolone carboxylic acids, cephalosporin compounds, and other rel related antibacterial compounds; and to novel processes for preparing these compounds and the salts thereof.

### Description of the Prior Art

In the process of developing novel medicinal compounds which possess a broad spectrum of potent anti-bacterial activities and are effective against various microorganisms, needs have existed for the discovery of novel intermediates capable of producing such compounds having improved antibacterial activities.

Japanese Patent Publication No. Hei 02-78659 discloses saturated azabicycloamine compounds whose molecular structure are bent for the reason that its rings are linked in cis form.

Consequently, the antibacterial compounds prepared therefrom did not exhibit sufficient antibacterial activity.

### Summary of the Invention

The present inventors have discovered that certain unsaturated azabicycloamine compounds which do not have stereoisomers relating to the form of their rings but have a planar molecular shape are useful for preparing various different antibacterial compounds such as cephalosporin and quinolone carboxylic acid derivatives with improved activities.

Accordingly, the present invention primarily pertains to providing novel azabicycloamine compounds useful as intermediate compounds for preparing such diverse and effective antibacterial compounds; and novel processes for the preparation thereof.

### Detailed Description of the Invention

The present invention relates to the azabicycloamine compounds, and their salts represented by the formula(I): 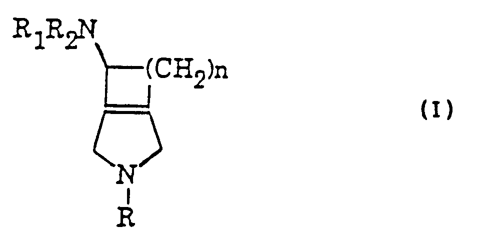
wherein:
R is a hydrogen atom or an amine protecting group;
R₁ and R₂, which may be the same or different, are hydrogen, optionally substituted lower alkyl, alkanoyl, optionally substituted benzoyl or naphthoyl, optionally substituted alkoxycarbonyl, or optionally substituted benzyloxycarbonyl; and
n is an integer from 1 to 4.

The amine protecting group of R in formula (I) may be benzenesulfonyl, paratoluenesulfonyl, methanesulfonyl, C₁₋₄-alkoxycarbonyl, benzyloxycarbonyl, paranitrobenzyloxycarbonyl, 2,2,2-trichlorethoxycarbonyl, C₁₋₂₀-alkanoyl, or optionally substituted benzoyl or naphthoyl; R₁ or R₂ may be optionally substituted lower alkyl, such as C₁₋₇-, preferably C₁₋₄-alkyl, especially methyl or ethyl, alkanoyl, preferably C₁₋₁₈-alkanoyl, including formyl, acetyl, propionyl, butanoyl, pentanoyl, hexanoyl, heptanoyl, octanoyl, decanoyl, undecanoyl, palmitoyl, oleoyl, stearoyl and cyclohexanoyl, optionally substituted benzoyl or naphthoyl, optionally substituted alkoxycarbonyl, preferably C₁₋₄-alkoxycarbonyl, such as ethoxycarbonyl, and 2,2,2-trichloroethoxycarbonyl, or optionally substituted benzyloxycarbonyl, such as paranitrobenzyloxycarbonyl.

Another aspect of the present invention relates to an acid addition salt of the compound of formula (I) wherein at least one nitrogen atom is basic nitrogen, which may include the salts of an inorganic acid such as hydrochloric, hydrobromic, phosphoric, or sulfuric acid, and of an organic acid such as formic, acetic, propionic, methane sulfonic, trichloroacetic, trifluoroacetic, lactic, maleic, malonic, fumaric, citric, tartaric, paratoluene sulfonic, benzene sulfonic, palmitic, oleic or stearic acid.

The process for preparing the compound of formula (Ia-1) may be summarized by the following Scheme(A): 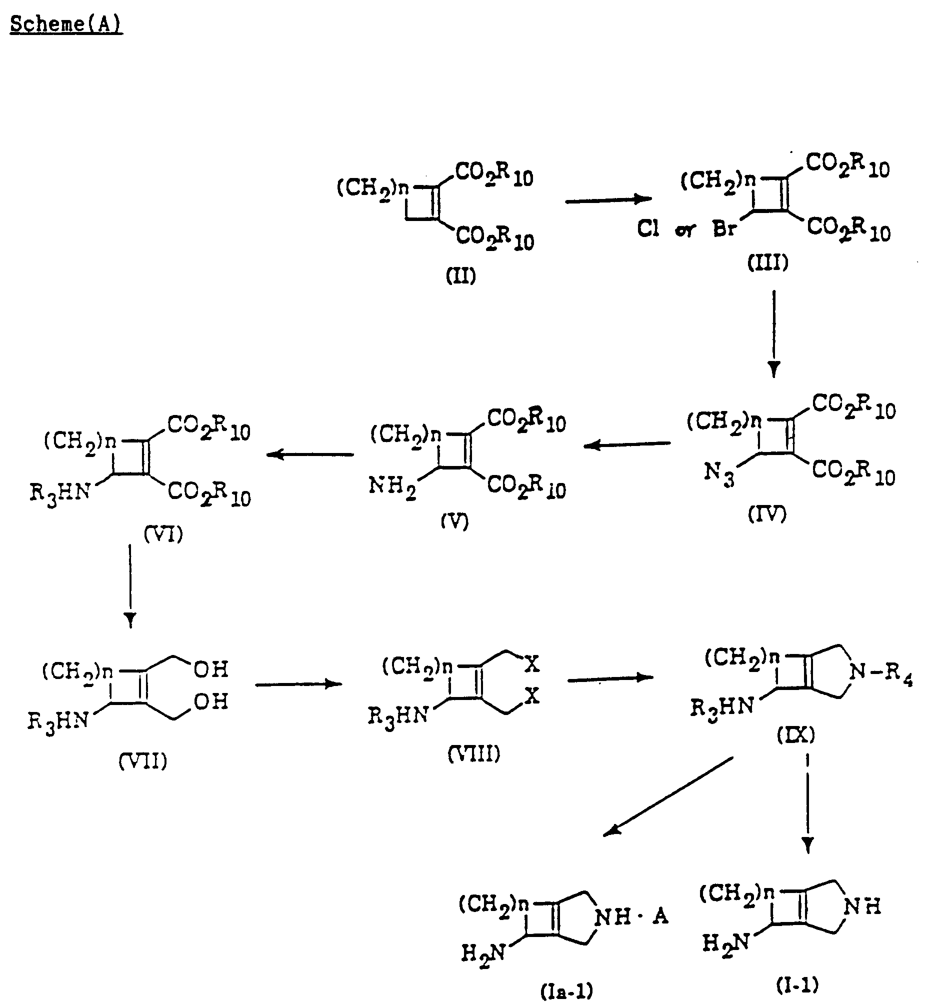
wherein:
n is an integer from 1 to 4;
R₁₀ is a C₁₋₄-alkyl group;
R₃ an amine protecting group, such as e.g. methoxycarbonyl, ethoxycarbonyl, t-butoxycarbonyl, 2,2,2-trichloroethoxycarbonyl, benzyloxycarbonyl, or paranitrobenzyloxycarbonyl;
R₄ has the same definition as R in formula (I), especially benzenesulfonyl, paratoluenesulfonyl, methanesulfonyl, or C_{₁₋₄}-alkoxycarbonyl;
X is iodine, bromine or chlorine, or a O-mesyl or O-tosyl grou; and
A is hydrochloric, hydrobromic, sulfonic or trifluoroacetic acid.

The compound of formula (IV) may be prepared by reacting sodium azide with the compound of formula (III) which may be prepared by heating the compound of formula (II) with N-bromo(or chloro) succinimide in the presence of a free radical-generating agent such as benzoyl peroxide or AIBN (aziisobutyronitrile).

The compound of formula (V) is prepared by reacting the compound of formula (IV) with triphenylphosphine in the presence of a solvent of tetrahydrofuran and water; and, thereafter, the compound of formula (VI) is prepared by reacting the compound of formula (V)with an amine protecting agent such as di-t-butyl dicarbonate or methyl(or ethyl-, trichloromethyl-, benzyl-or paranitrobenzyl-) chloroformate in the presence of base such as pyridine, triethylamine, sodium hydride, sodium hydroxide, or potassium carbonate.

The compound of formula (VI) is reduced with an ester reducing agent such as diisobutylaluminium hydride to obtain the alcohol of formula (VII), which is subsequently reacted with a halogenation agent such as phosphorous trichloride, phosphorous trifluoride, thionylchloride or oxalkyl chloride, or reacted with triphenylphosphine and carbon tetrachloride or carbon tetrabromide.

The compound of formula (IX) can be prepared by reacting the compound of formula (VIII) with an R₄ donating agent such as paratoluenesulfonamide, benzenesuffonamide, acetamide, benzamide, methanesulfonamide and urethane in the presence of a base such as sodium hydride, triethylamine or potassium carbonate.

The compound of formula (IX) is heated in hydrochloric acid, sulfuric acid, hydrobromic acid or trifluoroacetic acid to obtain the compound of formula (Ia-1); and may also be used for preparing the compound of formula (I-1) by removing the amine protecting group in the presence of a base such as sodium hydroxide or sodium ammonia.

The compound of formula (I) wherein R and R₁ are hydrogen and R₂ is lower alkyl (compound I-2, Ia-2) can be prepared in accordance with the following Scheme (B): 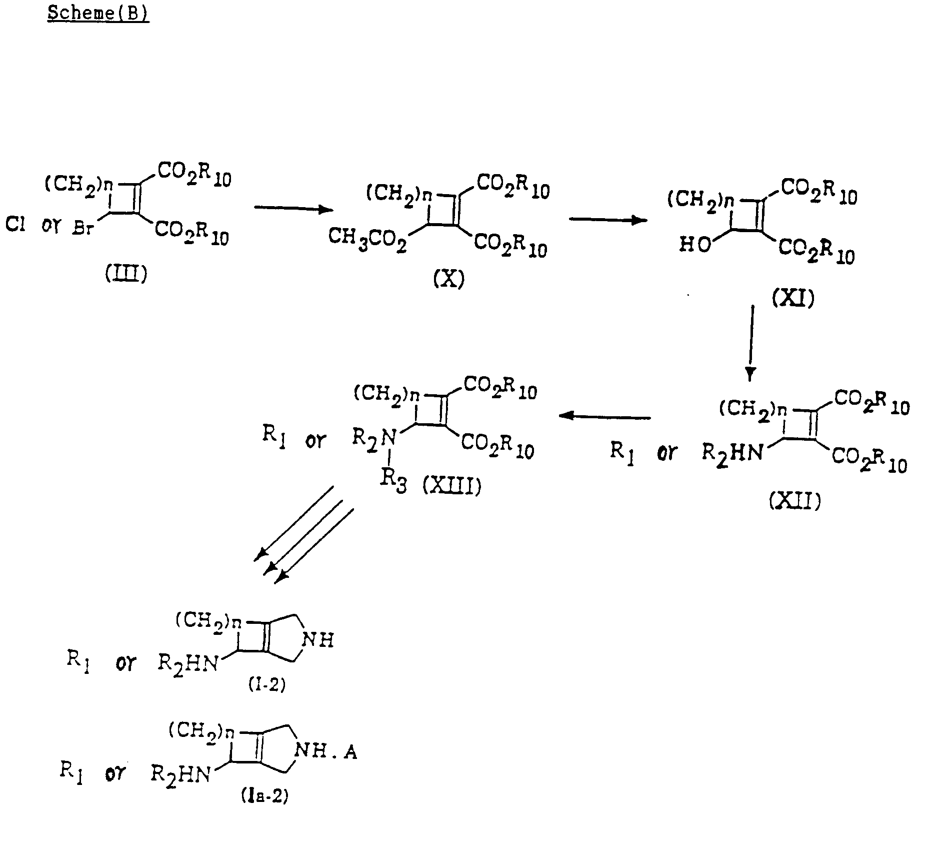
wherein:
n, R₁₀, R₂, R₃ and A have the same meanings as defined previously.

In Scheme(B), a compound of formula(X) is obtained by reacting the compound of formula(III) with sodium acetate or silver acetate, and a compound of formula(X) is subsequently deacetylated to give the compound of formula(XI).

The compound of formula(XI) is tosylated or mesylated, which subsequently is either reacted with a R₂ donating amine compound or undergoes a Mitsunobu reaction so as to produce a compound of formula(XII).

Protecting the nitrogen atom of formula(XII) with an amine protecting agent by employing the same method as used for the preparation of compound of formula(VI), the compound of formula(I-2) or (Ia-2) is prepared by repeating the process of preparing the compound of the formula(I-1) or (Ia-1).

Further, the compound of formula(I) wherein R and R₁ are hydrogen and R₂ is a lower alkyl group may be prepared by employing the method of Scheme (C) given below: 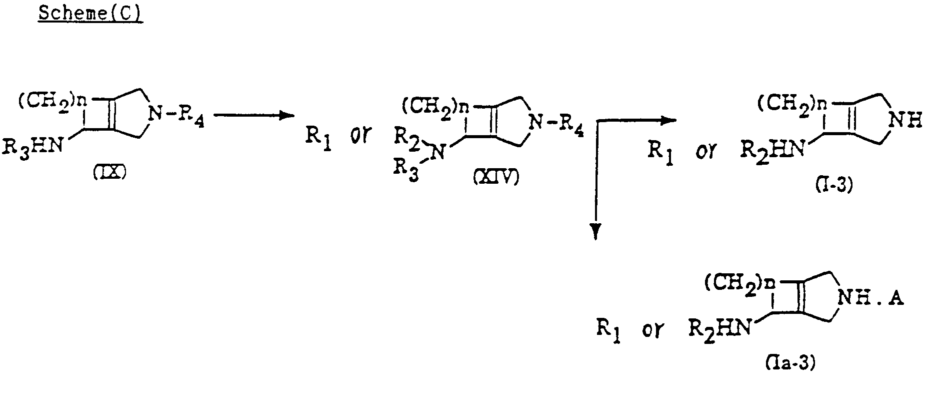
wherein:
n, R₂, R₃, R₄ and A have the same meanings as defined previously.

In Scheme(C), the compound of formula(IX) is reacted with an alkylhalide in the presence of a strong base such as sodium hydride to obtain the compound of formula(XIV), from which R₃ and R₄, the amine protecting groups are removed to produce the compound of formula(I-3) or (Ia-3).

Further, the compound of formula(I) wherein R₁ is a methyl group may be prepared by reducing the compound of formula (XV) in the presence of aluminium lithium hydride to obtain the compound of formula(XVI) and removing R₆ therefrom as shown in the following Scheme(D): 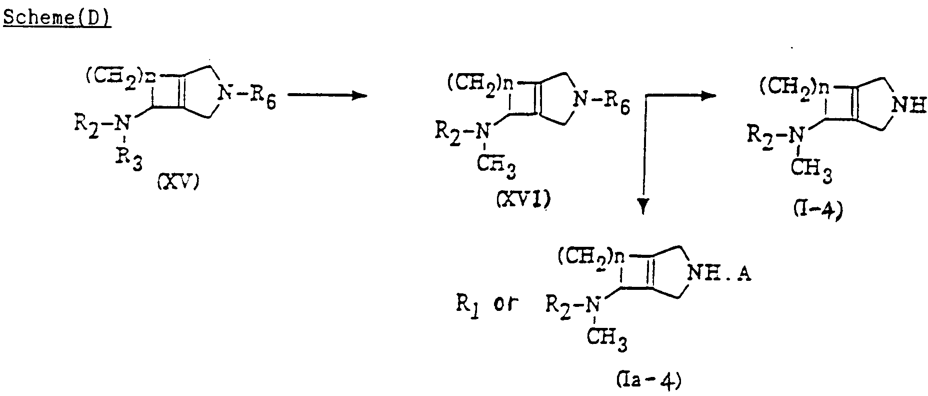
wherein:
n, A, R₂ and R₃ have the same meanings as previously defined; and
R₆ is a (para)toluenesulfonyl or methanesulfonyl group.

More specifically, the compound of formula(I), wherein one of R₁ and R₂ is a methyl group and the other is a hydrogen atom, can be prepared by employing the following Scheme(E): 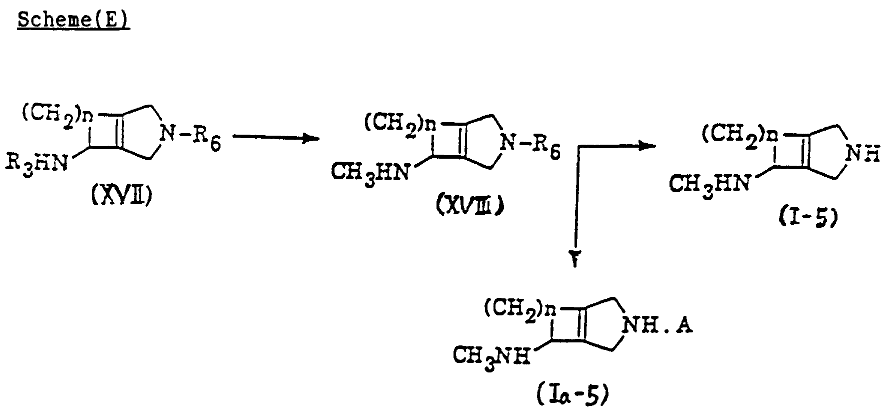
wherein:
n, A, R₃ and R₆ have the same meanings as previously defined; and
R₆ is a (para)toluenesulfonyl or methanesulfonyl group.

The compound of the formula(I-5) or (Ia-5) can be obtained by reducing R₃ in the compound of formula (XVII) to a methyl group in the presence of lithium aluminium hydride to give the compound of formula(XVIII) and by way of removing R₆ therefrom.

The following examples are intended to further illustrate the present invention, without limiting the scope of the invention.

### Example 1: Synthesis of 3-bromo-1-cyclopentene-1,2-dicarboxylic acid dimethylester

To a solution of 18g of 1-cyclopentene-1,2-dicarboxylic acid dimethylester dissolved in 180mℓ of carbon tetrachloride were added 17.4g of N-bromosuccinimide(NBS) and 1.6g of α ,α-azobis (isobutyronitrile)(AIBN); and the resultant mixture was refluxed for 5 hours. After filtering the reaction mixture, the filtrate was concentrated under a reduced pressure and purified by fractionating on the silica gel column chromatography to obtain 18.8g of the title compound in yellow oil(yield 73%).
- MS m/z(rel. int, %):: 263(M⁺, 43), 231(23), 183(100), 151(63)
- ¹H-NMR(CDCl₃, δ ppm):: 2.45(1H, m), 2.57(1H, m), 2.7 (1H, m), 3.05(1H, m), 3.73(6H, s), 5.25(1H, m)

### Example 2: Synthesis of 3-azido-1-cyclopentene-1,2-dicarboxylic acid dimethylester

To a solution of 18g of 3-bromo-1-cyclopentene-1,2-dicarboxylic acid dimethyl ester dissolved in 200mℓ of carbon tetrachloride was added a solution of 15.6g of sodium azide dissolved in 65mℓ of water; and the resultant solution was stirred for 16 hours. The reaction solution was refluxed for 1 hour and cooled; and the organic layer was separated. After the remaining aqueous layer was extracted twice with carbon tetrachloride, the organic layers were combined, dried over anhydrous magnesium sulfate and concentrated under a reduced pressure to obtain 13.4g of the title compound in yellow oil(yield 92%).
- MS m/z(rel. int, %):: 226(M⁺, 3.3), 187(67), 151(100), 138(45), 106(30), 93(27), 68(27)
- ¹H-NMR(CDCl₃, δ ppm):: 2.0(1H, m), 2.4(1H, m), 2.67(1H, m), 2.9(1H, m), 3.8(6H, s), 4.77(1H, m)

### Example 3: Synthesis of 3-amino-1-cyclopentene-1,2-dicarboxylic acid dimethylester

To a solution of 13.4g of 3-azido-1-cyclopentene-1,2-dicarboxylic acid dimethyl ester dissolved in 135mℓ of tetrahydro furan was added 15.5g of triphenylphosphine; and the resultant mixture was stirred for 16 hours under nitrogen gas.

After adding 1.6mℓ of water, the reaction mixture was stirred for 5 hours at a room temperature and concentrated under a reduced pressure; and the residue was dissolved in 250mℓ of dichloromethane. The reaction solution was extracted with 350mℓ of 2N hydrochloric acid, and, then, the aqueous layer was separated and neutralized with 15% aqueous NaOH solution.

The aqueous solution was extracted with 300mℓ of dichloromethane, dried over anhydrous magnesium sulfate and concentrated under a reduced pressure to obtain 5.9g of the title compound in pale yellow oil(yield 50%).
- MS m/z(rel. int, %);: 199(M⁺, 4), 184(3.5), 152(15), 140 (100), 108(28), 80(55)
- ¹H-NMR(CDCl₃, δ ppm):: 1.6(2H, m), 2.4(1H, m), 2.6(1H, m), 2.75(1H, m), 3.7(1H, m), 3.73(3H, s), 3.75(3H, s), 4.2(1H, m)

### Example 4: Synthesis of 3-(N-t-butoxycarbonyl)amino-1-cyclopentene-1,2-dicarboxylic acid dimethylester

To a solution of 5.9g of 3-amino-1-cyclpentene-1,2-dicarboxylic acid dimethylester dissolved in 80mℓ of methanol was added 7.1g of di-t-buthyl dicarbonate, and the resultant mixture was strirred for 16 hours at room temperature. The reaction mixture was concentrated under the reduced pressure and fractionated on silica gel column chromatography to obtain 8.42g of the title compound in white precipitate(yield 95%).
- MS m/z(rel. int, %):: 300(M⁺, 28), 244(88), 200(38), 183 (49), 167(53), 140(46), 57(100)
- ¹H-HMR(CDCl₃, δ ppm):: 1.4(9H, s), 1.73(1H, m), 2.8∼2.4 (3H, m), 3.74(3H, s), 3.76(3H, s), 4.76(1H, br.s), 5.03(1H, br.s)

### Example 5: Synthesis of 3- (N-t-butoxycarbonyl)amino-1,2-bis (hydroxymethyl)-1-cyclopentene

A solution of 18g of diisobutylaluminium hydride dissolved in 100mℓ of anhydrous tetrahydrofuran was cooled to -78°C and, then, a solution of 9.4g of 3-amino(N-t-butoxycarbonyl)amino-1-cyclopentene-1,2-dicarboxylic acid dimethyl ester dissolved in 90mℓ of tetrahydrofuran was added dropwise thereto for 1 hour. The resultant solution was stirred for 2 hours; and 9mℓ of methanol and 3mℓ of water were added carefully thereto.

The reaction mixture was extracted with 50% solution of methanol and dichloromethane(1:1, v/v) and concentrated under a reduced pressure to obtain 3.03g of the title compound in pale yellow oil(yield 40%).
- MS m/z(rel. int, %):: 243(M⁺)
- ¹H-NMR(CDCl₃, δ ppm):: 1.39(9H, s), 1.45(1H, m), 2.5∼2.25 (3H, m), 4.03(1H, m), 4.2(4H, m), 4.68(1H, m)

### Example 6: Synthesis of 3-(N-t-butoxycarbonyl)amino-1,2-bis (bromomethyl)-1-cyclopentene

To a solution of 3.03g of 3-amino(N-t-butoxycarbonyl)amino-1,2-bis(hydroxymethyl)-1-cyclopentene and 9.94g of carbon tetrabromide dissolved in 30mℓ of dichloromethane was added dropwise for 10 minutes a solution of 9.8g of triphenylphosphine dissolved in 20mℓ of dichloromethane, in a cold water bath. The resultant solution was stirred for 30 minutes in a cold water bath and concentrated under the reduced pressure; and the residue was dissolved in ethylether, which was filtered for the removal of undissolved solids. The filtrate was concentrated under a reduced pressure and purified by fractionating on the silica gel column chromatography to obtain 1.85g of the title compound in white precipitate(yield 40%).
- m.p. :: 104° ∼106°
- MS m/z(rel. int, %):: 369(M⁺), 312(50), 253(22), 234(90), 188(15), 152(100), 108(27), 91(23), 57(55)
- ¹H-NMR(CDCl₃, δ ppm):: 1.58(9H, s), 1.65(1H, m), 2.35∼2.65 (3H, m), 4.03(4H, m), 4.61(1H, br.s), 4.88(1H, br.s)

### Example 7: Synthesis of 3- (N-t-butoxycarbonyl)amino-1,2-bis (chloromethyl)-1-cyclopentene

To a solution of 3.03g of 3-amino(N-t-butoxycarbonyl)-1,2-bis(hydroxymethyl)-1-cyclopentene dissolved in 30mℓ of carbon tetrachloride was added 6.86g of triphenylphosphine; and the resultant mixture was refluxed for 3 hours. The reaction mixture was concentrated under a reduced pressure and fractionated on the silica gel column chromatography to obtain 1.21g of the title compound in white precipitate(yield 35%).
- m.p. :: 85° ∼88°
- MS m/z(rel. int, %):: 377(M⁺, 5), 222(85), 187(70), 163(50), 152(95), 127(27), 91(45), 57(100)
- ¹H-HMR(CDCl₃, δ ppm):: 1.39(9H, s), 1.6(1H, m), 2.4∼2.6 (3H, m), 4.1(4H, m), 4.5(1H, br.s), 4.8(1H, br.s)

### Example 8: Synthesis of 6- (N-t-butoxycarbonyl)amino-3-(paratoluenesulfonyl)-3-azabicyclo[3,3,0]oct-1(5)ene

To a solution of 1.85g of 3-amino(N-t-butoxycarbonyl) amino-1,2-bis(bromomethyl)-1-cyclopentene and 943mg of paratoluene sulfonamide dissolved in 20mℓ of dimethylformamide was added 500mg of sodium hydride(55∼60%) in a cold water bath. The resultant mixture was stirred for 5 hours; and 60mℓ of water was added thereto. The reaction solution was, then extracted with dichloromethane, concentrated under a reduced pressure and purified by fractionating on the silica gel column chromatography to obtain 950mg of the title compound in white precipitate(yield 50%).
- m.p. :: 159° ∼160°C
- MS m/z(rel. int, %):: 378(M⁺, 0.5), 321(18), 260(95), 234 (20), 167(100), 155(17), 106(93), 91 (27), 80(18), 57(17)
- ¹H-NMR(CDCl₃, δ ppm):: 1.5(9H, s), 1.88(1H, m), 2.09 (1H, m), 2.20(1H, m), 2.44(3H, s), 2.69(1H, s), 3.99(4H, br.s), 4.45 (1H, br.m), 4.56(1H, br.s), 7.31(2H, d, J=8Hz), 7.73(2H, d, J=8Hz)

### Example 9: Synthesis of 6-amino-3-azabicyclo[3,3,0]oct-1(5)ene dihydrobromide

To a solution of 760mg of 6-amino(N-t-butoxycarbonyl)amino-3-(paratoluenesulfonyl)-3-azabicyclooct-1(5)ene dissolved in 2.6mℓ of 30% aqueous hydrobromide was added 430mg of phenol. The resultant mixture was refluxed for 5 hours; and 60mℓ of water was added thereto. The reaction mixture was washed with chloroform until any color did not remain in the chloroform layer. The aqueous layer was separated, decolorized by the filtration through by using active carbon, and concentrated under a reduced pressure. The residue was suspended in a solvent of ethanol and ehtylether(1:2, v/v) to give 330mg of the title compound in white precipitate(yield 65%).
- m.p. :: 195° ∼198°C
- MS m/z(rel. int, %):: 124(M⁺, 7), 107(100), 95(33), 82(53), 80(90)
- ¹H-NMR(CDCl₃, δ ppm):: 2.1∼2.8(4H, m), 3.95(2H, s), 3.96 (2H, s), 4.29(1H, m)

### Example 10: Synthesis of 6-methylamino-3-(paratoluenesulfonyl)-3-azabicyclo[3,3,0]-oct-1(5)ene

To a solution of 100mg of 6-amino(N-t-butoxycarbonyl)-3-(paratoluenesulfonyl)-3-azabicyclo[3,3,0]oct-1(5)ene dissolved in 3mℓ of tetrahydrofuran was added 23mg of lithium aluminium hydride. The resultant mixture was refluxed for 20 minutes; and 10mℓ of methanol and 0.1mℓ of water were added thereto. The reaction mixture was extracted with 50% solution of methanol/dichloromethane(1:1, v/v), concentrated under a reduced pressure and purified by fractionating on the silica gel column chromatography to obtain 70mg of the title compound in white precipitate(yield 83%).
- MS m/z(rel, int, %):: 292(M⁺)
- ¹H-NMR(CDCl₃, δ ppm):: 1.89(1H, m), 2.13(1H, m), 2.23(1H, br.s), 2.24(3H, s), 2.46(1H, m), 2.52 (3H, s), 3.66(1H, br.m), 3.84∼4.16 (4H, m), 7.27(2H, d, J=8Hz), 7.63(2H, d, J=8Hz)

### Example 11: Synthesis of 6-methylamino-3-azabicyclo[3,3,0]oct-1(5) ene dihydrobromide

A solution of 70mg of 6-methylamino-3-(paratoluenesulfonyl)-3-azabicyclo[3,3,0]oct-1(5)ene suspended in 0.2mℓ of hydrogen bromide was refluxed for 5 hours; and 10mℓ of water was added thereto. The reaction mixture was washed with chloroform until any color did not remain in the chloroform layer. The aqueous layer was separated, decolorized by being filtered through active carbon, and concentrated under a reduced pressure. The residue was suspended in a solvent of ethanol and ethylether(1:2, v/v) to obtain 40mg of the title compound in white precipitate(yield 55%).
- MS m/z(rel. int, %):: 138(M⁺, 7), 107(100), 94(18), 80(55), 68(12)
- ¹H-NMR(CDCl₃, δ ppm):: 2.2∼2.8(4H, m), 2.51(3H, s), 3.96 (4H, m), 4.22(1H, br.m)

### Example 12: Synthesis of 6-(N-t-butoxycarbonyl-N-ethyl)amino-3-(paratoluenesulfonyl)-3-azabicyclo[3,3,0]oct-1(5)ene

To a solution of 1.89g of 6-amino(N-t-butoxycarbonyl)-3-(paratoluenesulfonyl)-3-azabicyclo[3,3,0]oct-1(5)ene dissolved in 20mℓ of dimethylformamide in nitrogen environment was added 280mg of sodium hydride. The resultant mixture was stirred for 2 hours at room temperature; and 850mg of ethyliodide was added thereto. The reaction mixture was stirred for 5 hours, concentrated under a reduced pressure and purified by fractionating on the silica gel column to obtain 1.73g of the title compound(yield 85%).
- ¹H-NMR(CDCl₃, δ ppm):: 1.45(3H, t, J=7Hz), 1.89∼2.05 (2H, m), 2.21(1H, m), 2,46(3H, s), 270(1H, m), 4.01(4H, br.s), 4.20(2H, q, J=7Hz), 4.47(1H, m), 7.30(2H, d, J=8Hz), 7.74(2H, d, J=8Hz)

### Example 13: Synthesis of 6-ethylamino-3-azabicyclo[3,3,0]oct-1(5)-enedihydrobromide

406mg of 6-(N-t-butoxycarbonyl-N-ethyl)amino-3-(paratoluenesulfonyl)-3-azabicyclo[3,3,0]oct-1(5)ene and 100mg of phonol were suspended in 10mℓ of 30% hydrogen bromide solution. The resultant mixture was refluxed for 5 hours, cooled and washed 5 times with 20mℓ of chloroform. The aqueous solution was decolorized by using active carbon, filterted and concentrated. The residue was solidified in a mixture solution of ethanol and ethylether(1:2 v/v) to obtain 200mg of the title compound in white precipitate(yield 63%).
- m.p.:: 185° ∼188°C
- ¹H-NMR(CDCl₃, δ ppm):: 1.40(3H, t, J=7Hz), 2.50∼2.81 (4H, m), 3.94(4H, br.s), 4.18(2H, q, J=7Hz), 4.24(1H, m)

### Example 14: Synthesis of 6-(N-ethyl-N-methyl)amino-3-(paratoluenesulfonyl)-3-azabicyclo[3,3,0]oct-1(5)ene

To a solution of 400mg of 6-(N-t-butoxycarbonyl-N-ethyl) amino-3-(paratoluenesulfonyl)-3-azabicyclo[3,3,0]oct-1(5)ene dissolved in 10mℓ of tetrahydrofuran was added 100mg of lithium aluminium hydride. The resultant mixture was refluxed for 25 minutes; and 10mℓ of methanol and 0.5mℓ of water were added thereto.

After the reaction mixture was filtered, the filtrate was concentrated under a reduced pressure and purified by fractionating on the silica gel column to obtain 250mg of the title compound (yield 75%)
- ¹H-NMR(CDCl₃, δ ppm):: 1.42(3H, t, J=7Hz), 1.90(1H, m), 2.17∼2.20(2H, m), 2.23(3H, s), 2.43 (1H, m), 2.53(3H, s), 3.71(1H, m), 3.95(4H, br.s), 4.24(2H, q, J=7Hz), 7.26(2H, d, J=8Hz), 7.64(2H, d, J=8Hz)

### Example 15: Synthesis of 6-(N-ethyl-N-methyl)amino-3-azabicyclo [3,3,0]oct-1(5)ene dihydrobromide

160mg of 6-(N-ethyl-N-methyl)amino-3-(parotoluenesulfonyl)-3-azabicyclo[3,3,0]oct-1(5)ene was suspended in 5mℓ of 30% hydrobromide solution; and the resultant mixture was refluxed for 5 hours followed by addition of 10mℓ of water. The reaction mixture was washed with chloroform; and the aqueous layer was separated, decolorized by being filtered through active carbon and concentrated under a reduced pressure. The residue was suspended in 20mℓ of a mixture of ethanol and ethylether(1:2, v/v); and the precipitates produced therefrom subsequently filtered and dried to obtain 123mg of the title compound(yield 75%).
- m.p.:: 165° ∼168°C
- ¹H-NMR(D₂O, δ ppm):: 1.42(3H, t, J=7Hz), 2.25(3H, s), 2.51∼2.80(4H, m), 3.90(4H, br.s), 4.15(2H, q, J=7Hz), 4.21(1H, m)

### Example 16: Synthesis of 3-bromo-1-cyclohexene-1,2-dicarboxylic acid dimethylester

To a solution of 18g of 1-cyclohexene-1,2-dicarboxylic acid dimethylester dissolved in 180mℓ of carbon tetrachloride were added 16.1g of N-bromosuccinimide and 1.49g of α ,α -azobis (isobutyronitrile); and the resultant mixture was refluxed for 5 hours. The reaction mixture was filtered, concentrated under a reduced pressure and fractionated on the silica gel column chromatography to obtain 17.6g of the title compound(yield 70%).
- MS m/z(rel. int, %):: 277(M⁺)
- ¹H-NMR(CDCl₃, δ ppm):: 5.23(1H, m), 3.81(6H, s), 3.1(1H, m), 2.7∼2.4(3H, m), 1.51(2H, m)

### Example 17: Synthesis of 3-azido-1-cyclohexene-1,2-dicarboxylic acid dimethylester

To a solution of 17g of 3-bromo-1-cyclohexene-1,2-dicarboxylic acid dimethylester dissolved in 180mℓ of carbon tetrachloride was added a solution of 14g of sodium azide dissolved in 55mℓ of water; and the resultant mixture was stirred for 16 hours at room temperature. The reaction mixture was refluxed for 1 hour and cooled; and the organic layer was separated, and the remaining aqueous layer was extracted twice with carbon tetrachloride. All the organic layers were conbined, dried over anhydrous magnesium sulfate, and concentrated under a reduced pressure to obtain 13.4g of the title compound in yellow oil(yield 91%).
- MS m/z(rel. int, %):: 240(M⁺)
- ¹H-NMR(CDCl₃, δ ppm):: 4.79(¹H, m), 3.79(6H, s), 2.9(1H, m), 2.7(1H, m), 2.41(1H, m), 2.1 (1H, m), 1.45(2H, m)

### Example 18: Synthesis of 3-amino-1-cyclohexene-1,2-dicarboxylic acid dimethyl ester

To a solution 13.4g of 3-azido-1-cyclohexene-1,2-dicarboxylic acid dimethylester dissolved in 135mℓ of tetrahydrofuran was added 14.5g of triphenylphosphine. The resultant mixture was stirred for 16 hours under nitrogen gas; and 1.55mℓ of water was added thereto. The reaction mixture was further stirred for 5 hours at a room temperature and concentrated under a reduced pressure; and the residue was dissolved in 240mℓ of dichloromethane. The solution was extracted with 350mℓ of 2N hydrochloric acid solution; and the aqueous layer was separated therefrom and neutralized with 15% NaOH solution. The neutralized solution was extracted with 300mℓ of dichloromethane, dried over anhydrous magnesium sulfate and concentrated under a reduced condition to obtain 5.7g of the title compound in pale yellow oil(yield 48%).
- MS m/z(rel. int, %):: 213(M⁺)
- ¹H-NMR(CDCl₃, δ ppm):: 4.21(1H, m), 3.73(3H, s), 3.72(3H, s), 3.56(1H, m), 2.75(1H, m), 2.6 (1H, m), 2.41(1H, m), 1.49(2H, m)

### Example 19: Synthesis of 3-(N-t-butoxycarbonyl)amino-1-cyclohexene-1,2-dicarboxylic acid dimethylester

To a solution of 5.7g of 3-amino-1-cyclohexene-1,2-dicarboxylic acid dimethylester dissolved in 80mℓ of methanol was added 6.4g of di-t-butylcarbonate. The resultant mixture was stirred for 16 hours at a room temperature, concentrated under a reduced pressure and fractionated on the silica gel column chromatography to obtain 7.95g of the title compound in white precipitate (yield 95%).
- MS m/z(rel. int, %):: 314(M⁺)
- ¹H-NMR(CDCl₃, δ ppm):: 5.09(1H, br.s), 4.71(1H, br.s), 3.74(3H, s), 3.73(3H, s), 2.75(1H, m), 2.61(1H, m), 2.43(1H, m), 1.48(2H, m), 1.43(9H, s)

### Example 20: Synthesis of 3-(N-t-butoxycarbonyl)amino-1,2-bis (hydroxymethyl)-1-cyclohexene

14.4g of diisobutylaluminium hydride was dissolved in 90mℓ of anhydrous tetrahydrofuran; and the solution was cooled to -78°C. Thereafter, a solution of 7.9g of 3-amino(N-t-butoxycarbonyl)amino-1-cyclohexene-1,2-dicarboxylic acid dimethylester dissolved in 80mℓ of tetrahydrofuran was added dropwise to the cooled solution for 1 hour, which was stirred for 16 hours at a room temperautre. 8mℓ of methanol and 2.7mℓ of water were added carefully to the reaction mixture, which was extracted with 200mℓ of 50% solution of methanol/dichloromethane(1:1, v/v) and concentrated under a reduced pressure to obtain 2.78g of the title compound in pale yellow oil (yield 43%).
- MS m/z(rel. int, %):: 257(M⁺)
- ¹H-NMR(CDCl₃, δ ppm):: 4.69(1H, br.s), 4.31∼4.05(5H, m), 2.55∼2.25(3H, m), 4.31∼4.05(5H, m), 2.55∼2.25(3H, m), 1.45(2H, m), 1.41 (9H, s)

### Example 21: Synthesis of 3-(N-t-butoxycarbonyl)amino-1,2-bis (bromomethyl)-1-cyclohexene

To a solution of 2.7g of 3-amino(N-t-butoxycarbonyl)amino-1,2-bis(hydroxymethyl)-1-cyclohexene and 8.35g of carbon tetrabromide dissolved in 25mℓ of dichloromethane was added dropwise a solution of 8.1g of triphenylphosphine dissolved in 20mℓ of dichloromethane, in a cold water bath. The resutant solution was stirred for 30 minutes in a cold water bath and concentrated under a reduced pressure; the residue was dissolved in ethylether and filtered to remove undissolved solids; and the filtrate was concentrated under a reduced pressure and fractionated on the silica gel column chromatography to obtain 1.65g of the title compound in white precipitate (yield 41%).
- MS m/z(rel. int, %):: 383(M⁺)
- ¹H-HMR(CDCl₃, δ ppm):: 4.90(1H, br.s), 4.62(1H, br.s), 4.01(4H, m), 2.7∼2.35(3H, m), 1.75∼ 1.45(3H, m), 1.43(9H, s)

### Example 22: Synthesis of 6-(N-t-butoxycarbonyl)amino-3-(paratoluenesulfonyl)-3-azabicyclo[4,3,0]non-1(5)ene

To a solution of 1.6g of 3-amino(N-t-butoxycarbonyl)amino-1,2-bis(bromomethyl)-1-cyclohexene and 785mg of paratoluenesulfonamide dissolved in 15mℓ of dimethylformamide was added 416mg of sodium hydride(55~60%) in a cold water bath; and the resultant mixture was stirred for 5 hours. After 60mℓ of water is added, the reaction mixture was extracted with dichloromethane, concentrated in a reduced pressure and fractionated on the silica gel column chromatography to obtain 767mg of the title compound in white precipitate(yield 47%).
- MS m/z(rel. int, %):: 392(M⁺)
- ¹H-NMR(CDCl₃, δ ppm):: 4.51(1H, br.s), 4.85(1H, br.s), 4.1∼3.85(4H, m), 2.61(1H, m), 2.21 (1H, m), 2.11(1H, m), 1.73(1H, m), 1.45(2H, m), 1.39(9H, s)

### Example 23: Synthesis of 6-amino-3-azabicyclo[4,3,0]non-1(5)ene dihydrobromide

To a solution of 760mg of 6-amino(N-t-butoxycarbonyl)amino-3-(paratoluenesulfonyl)-3-azabicyclo[4,3,0]non-1(5)ene suspended in 2.5mℓ of 30% hydrobormide solution was added 410mg of phenol; and the resultant mixture was refluxed for 5 hours. After 40mℓ of water was added, the reaction mixture was washed with chloroform until any color did not remain in the chloroform layer; and the aqueous layer was filtered through active carbon, concentrated under a reduced pressure and precipitated in 30mℓ of ethanol/ethylether (1:2, v/v) solution to obtain 408mg of the title compound in white precipitate(yield 70%).
- MS m/z(rel. int, %):: 137(M⁺)
- ¹H-NMR(CDCl₃, δ ppm):: 4.29(1H, br.s), 3.95(4H, s), 2.79 (1H, m), 2.45(1H, m), 2.35(1H, m), 2.22(1H, m), 1.48(2H, m)

### Preparation Example 1: Preparation of 7-{6-amino-3-azabicyclo[3,3,0] oct-1(5)ene-3-yl}-1-cyclopropyl-6,8-difluoro-1,4-dihydro-4-oxoquinoline-3-carboxylic acid (Compound A)

To a mixture of 330mg of 6-amino-3-azabicyclo[3,3,0]oct-1(5)ene dihydrobromide and 320mg of 1-cyclopropyl-6,7,8-trifluoro-1,4-dihydro-4-oxoquinoline-3-carboxylic acid suspended in 10mℓ of acetonitrile was added 616µℓ of 1,8-diazabicyclo[5,4,0]undec-7-ene; and the resultant mixture was stirred for 5 hours. The reaction mixture was cooled and filtered; and the residue was washed with acetonitrile to obtain 340mg of the title compound in pale yellow precipitate(yield 85%).
- m.p.:: 213° ∼215°C
- MS m/z(rel. int, %):: 387(M⁺, 20), 370(100), 343(17), 326 (73), 199(25)
- ¹H-NMR(CDCl₃ + CD₃COOD, δ ppm):: 1.26(4H, m), 2.35(1H, m), 2.52(1H, m), 2.61(1H, m), 2.90 (1H, m), 4.1(1H, m), 4.39(1H, m), 4.63(4H, br.s), 7.76(1H, dd, J=14.7, 1.5Hz), 8.75 (1H, s)

### Preparation Example 2: Preparation of 7-{6-amino-3-azabicyclo[4,3,0] non-1(5)ene-3-yl}-1-cyclopropyl-6,8-difluoro-1,4-dihydro-4-oxoquinoline-3-carboxylic acid (Compound B)

To a mixture of 400mg of 6-amino-3-azabicyclo[4,3,0]non-1(5)ene dihydrobromide and 365mg of 1-cyclopropyl-6,7,8-trifluoro-1,4-dihydro-4-oxoquinoline-3-carboxylic acid suspended in 10mℓ of acetonitrile was added 735µℓ of 1,8-diazabicyclo[5,4,0]undec-7-ene; and the resultant mixture was refluxed for 5 hours. The reaction mixture was cooled and filtered; and the residue was washed with acetonitrile to obtain 387mg of the title compound in pale yellow precipitate(yield 75%).
- MS m/z(rel. int, %):: 401(M⁺)
- ¹H-NMR(CDCl₃ + CD₃COOD, δ ppm):: 8.76(1H, s), 7.73(1H, d, J=13Hz), 4.68(1H, br.s), 4.64(4H, br.s), 4.42(1H, br.s), 4.10(4H, br.s), 2.91 (1H, m), 2.65(1H, m), 2.55 (1H, m), 2.35(1H, m), 1.47 (2H, m), 1.25(4H, m)

### In vitro anti-bacterial activity test

Quinolone compounds may be prepared by using a compound of formula(I) as shown in Preparation Examples. In order to demonstrate the effectiveness of the quinolone compound prepared by using a compound of the present invention, the minimal inhibitory concentration(MIC)s of compounds A and B was determined and compared with that of ciprofloxacin. These MIC values were taken by employing two-fold dilution method; that is, two-fold aerial dilutions of each of the test compounds were made and dispersed in a Mueller-Hinton agar medium; 2µℓ of the standard strain which had the 10⁷ CFU(colony forming unit) per mℓ was inoculated on the medium; and these were incubated at 37°C for 20 hours. The result of the MIC tests are shown in Table 1.

**Table 1**

| In vitro anti-bacterial activity test(MIC, µg/mℓ) | | | |
|---|---|---|---|
| Strain | Compound A | Compound B | Ciprofloxacin |
| Streptococcus pyogenes 308 | 0.195 | 0.195 | 3.125 |
| Streptococcus pyogenes 77 | 0.098 | 0.098 | 1.563 |
| Streptococcus faecium MD 8b | 0.098 | 0.098 | 0.781 |
| Staphylococcus aureus SG 511 | 0.025 | 0.049 | 0.195 |
| Staphylococcus aureus 285 | 0.049 | 0.049 | 0.781 |
| Staphylococcus aureus 503 | 0.049 | 0.049 | 0.781 |
| Escherichia coli 055 | 0.013 | 0.013 | 0.013 |
| Escherichia coli DC 0 | 0.391 | 0.391 | 0.195 |
| Escherichia coli DC 2 | 0.098 | 0.098 | 0.098 |
| Escherichia coli TEM | 0.049 | 0.098 | 0.025 |
| Escherichia coli 1507E | 0.049 | 0.049 | 0.025 |
| Pseudomonas aeruginosa 9027 | 0.781 | 1.563 | 0.391 |
| Pseudomonas aeruginosa 1592E | 0.781 | 0.781 | 0.195 |
| Pseudomonas aeruginosa 1771 | 0.781 | 0.781 | 0.391 |
| Pseudomonas aeruginosa 1771M | 0.391 | 0.781 | 0.098 |
| Salmonella typhimurium | 0.049 | 0.049 | 0.025 |
| Klebsiella aerogenes 1082E | 0.049 | 0.049 | 0.013 |
| Klebsiella aerogenes 1522E | 0.098 | 0.049 | 0.025 |
| Enterobacter cloacae P 99 | 0.025 | 0.013 | 0.025 |
| Enterobacter cloacae 132E | 0.025 | 0.025 | 0.025 |

## Claims

1. An azabicycloamine of formula (I) and the salts thereof: 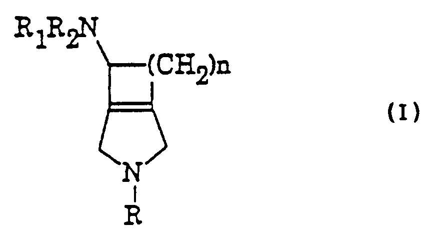 wherein
R is hydrogen or an amine protecting group;
R₁ and R₂, which may be the same or different, are hydrogen, optionally substituted lower alkyl, alkanoyl, optionally substituted benzoyl or naphthoyl, optionally substituted alkoxycarbonyl, or optionally substituted benzyloxycarbonyl; and
n is an integer from 1 to 4.

2. An azabicycloamine of claim 1 and the salts thereof, wherein the amine protecting group in R is benzenesulfonyl, paratoluenesulfonyl, methanesulfonyl, C₁₋₄ alkoxycarbonyl, benzyloxycarbonyl, paranitrobenzyloxycarbonyl, 2,2,2-trichlorethoxycarbonyl, C₁₋₂₀ alkanoyl, or optionally substituted benzoyl or naphthoyl.

3. A compound of claim 1 wherein R, R₁ and R₂ are hydrogen, as represented by the formula (I-1), and the acid addition salts thereof as represented by formula (Ia-1): 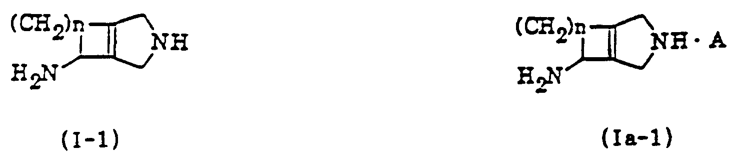 wherein A is a hydrochloric acid, hydrobromic acid, sulfuric acid or trifluoroacetic acid.

4. A compound of claim 1 wherein R and R₁ are hydrogen atoms; and R₂ is optionally substituted lower alkyl, alkanoyl, optionally substituted benzoyl or naphthoyl, optionally substituted alkoxycarbonyl, or optionally substituted benzyloxycarbonyl, as represented by formula (I-2), and the addition salts thereof as represented by formula (Ia-2): 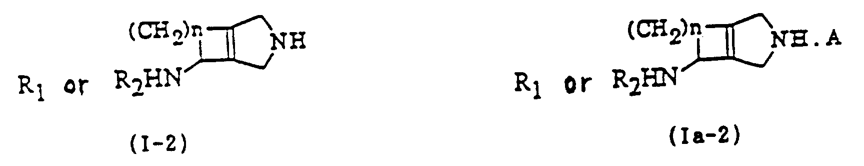 wherein A is hydrochloric, hydrobromic, sulfuric or trifluoroacetic acid.

5. A compound of claim 1 and the salts thereof wherein n is 2 or 3.

6. A salt of claim 1 which is an acid addition salt, wherein the acid of the acid addition salt is an inorganic acid selected from the group consisting of hydrochloric, hydrobromic, sulfuric and phosphoric acid, or an organic acid selected from the group consisting of formic, acetic, propionic, methane sulfonic, trichloroacetic, trifluoroacetic, lactic, maleic, malonic, fumaric, citric, tartaric, paratoluenesulfonic, benzenesulfonic, palmitic, oleic and stearic acid.

7. A process for preparing a compound of formula (Ia-1) of claim 3, which comprises:
(a) heating a compound of formula (II) with N-bromo (or chloro) succinimide in the presence of a free radical-generating agent to obtain a compound of formula (III);
(b) reacting the compound of formula (III) with an azide to obtain a compound of formula (IV);
(c) reacting the compound of formula (IV) with triphenylphosphine in a solvent to obtain a compound of formula (V);
(d) reacting the compound of the formula (V) with an amine protecting agent to obtain a compound of formula (VI);
(e) reducing the compound of formula (VI) with a reducing agent to obtain a compound of formula (VII);
(f) reacting the compound of the formula (VII) with a halogenation agent selected from the group consisting of phosphorous trichloride, phosphorous trifluoride, thionylchloride and oxalylchloride; or with triphenylphosphine and with carbon tetrachloride or carbon tetrabromide to obtain a compound of the formula (VIII);
(g) reacting the compound of formula (VIII) with an R₄ donating agent selected from the group consisting of paratoluenesulfonamide, benzenesulfonamide, acetamide, benzamide, methanesulfonamide and urethane in the presence of a base to obtain a compound of formula (IX); and
(h) heating the compound of formula (IX) in an acid selected from the group consisting of hydrochloric, sulfuric hydrobromic and trifluoroacetic acid: 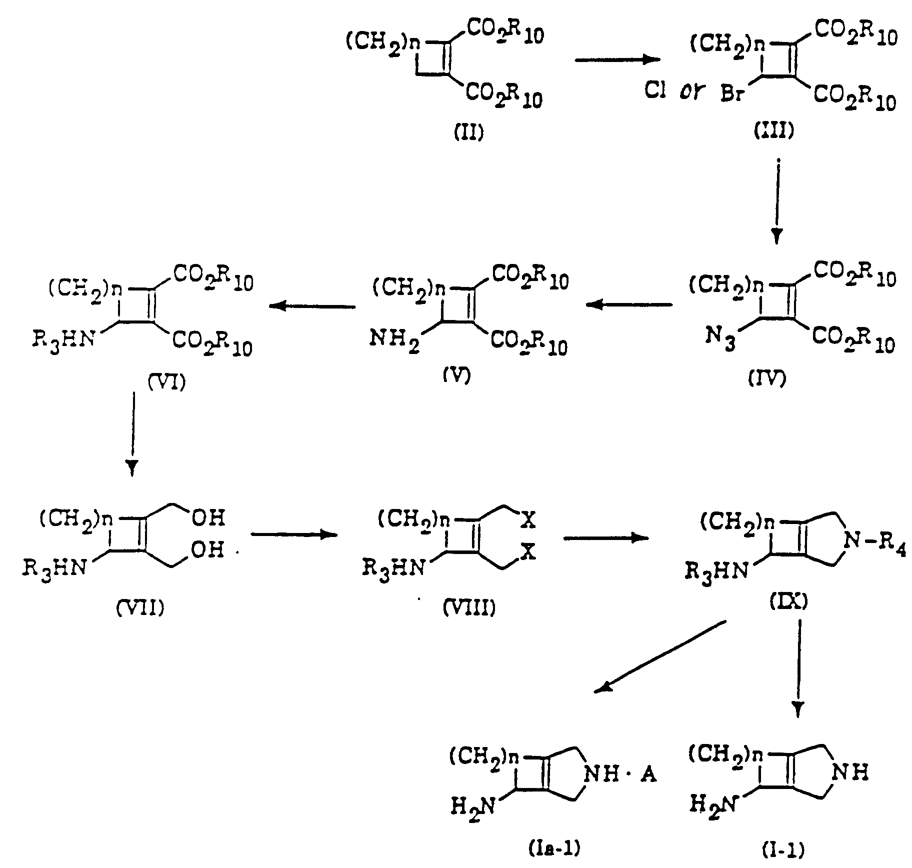 wherein:
n is an integer from 1 to 4;
R₁₀ is a C₁₋₄ alkyl;
R₃ is an amine protecting group;
R₄ is a hydrogen atom or an amino protecting group;
X is an iodine, bromine or chlorine atom, or O-mesyl or O-tosyl group; and
A is hydrochloric, hydrobromic, sulfonic or trifluoroacetic acid.

8. The process of claim 7, wherein R₃ is methoxycarbonyl, ethoxycarbonyl, t-butoxycarbonyl, 2,2,2-trichloroethoxycarbonyl, benzyloxycarbonyl and paranitrobenzyloxycarbonyl.

9. The process of claim 7, wherein the amino protecting group is benzenesulfonyl, paratoluene sulfonyl, methanesulfonyl or lower alkoxycarbonyl.

10. The process of claim 7, wherein the free radical-generating agent is benzoylperoxide or azaisobutyronitrile.

11. The process of claim 7, wherein the amine protecting agent is selected from the group consisting of di-t-butyl dicarbonate or methyl-, ethyl-, trichloromethyl-, benzyl- or paranitrobenzyl chloroformate in the presence of pyridine, sodium hydride, potassium carbonate, triethyl amine, sodium hydroxide.

12. A process for preparing a compound of formula (I-1), which comprises removing the nitrogen protecting group in the presence of a base.

13. The process of claim 12, wherein the base is sodium hydroxide or sodium ammonia.

14. A process for preparing a compound of formula (I-2) or (Ia-2), which comprises:
(a) reacting a compound of formula (III) with sodium acetate or silver acetate to obtain a compound of formula (X);
(b) deacetylating the compound of formula (X) to obtain a compound of formula (XI);
(c) mesylating or tosylating the compound of formula (XI) and reacting with a R₂ donating amine compound; or reacting the compound of formula (XI) by employing Mitsunobu reaction to protect the amine in a compound of the formula (XII); and
(d) following the same procedures as in steps of (e), (f), (g) and (h) of claim 7: 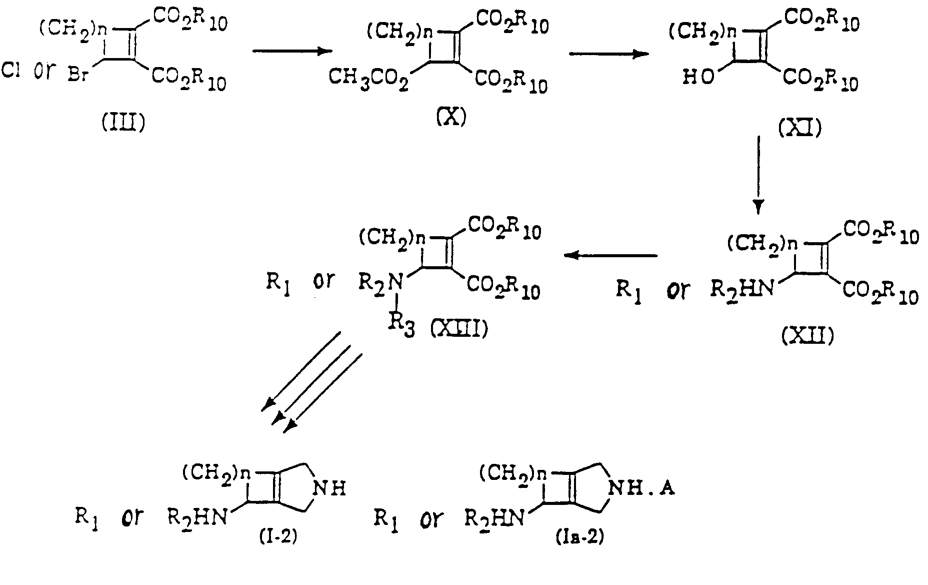 wherein
n, R, R₂, R₃ and A have the same meanings as defined above.

15. A process for preparing a compound of formula (I-3) or (Ia-3), which comprises reacting a compound of formula (IX) with an alkylhalide in the presence of a strong base to obtain a compound of formula (XIV), from which R₂ and R₄ the amino protecting groups, are removed to produce the compound of formula (I-3) or (Ia-3) 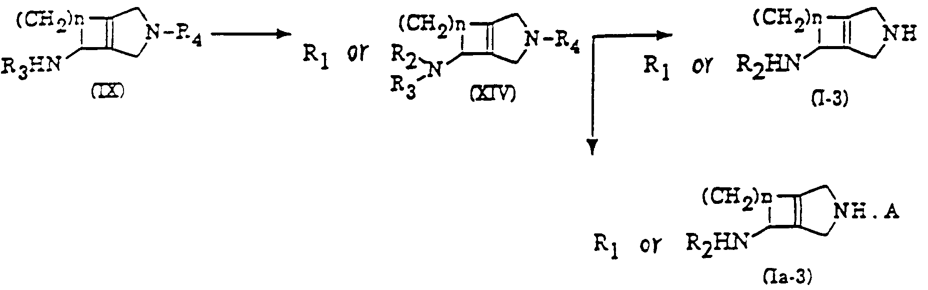 wherein n, R₂, R₃, R₄ and A are as defined above.

16. A process for preparing a compound of formula (I-4) or (Ia-4), which comprises reducing a compound of formula (XV) in the presence of aluminum lithium hydride to obtain a compound of formula (XVI) and removing the protecting group R₆ 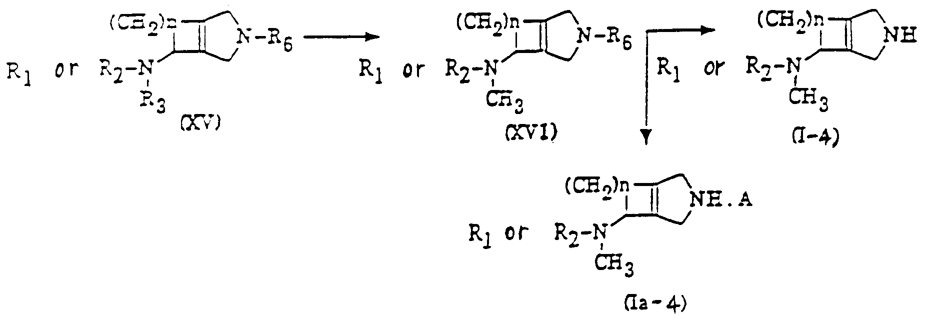 wherein n, A, R₂ and R₃ are as defined above and R₆ is p-toluenesulfonyl or methanesulfonyl.

17. A rpocess for preparing a compound of formula (I-5) or (Ia-5), which comprises reducing R₃ in the compound of formula (XVII) to a methyl group in the presence of lithium aluminum hydride to give the compound of formula (XVIII) and then removing the protecting group R₆ 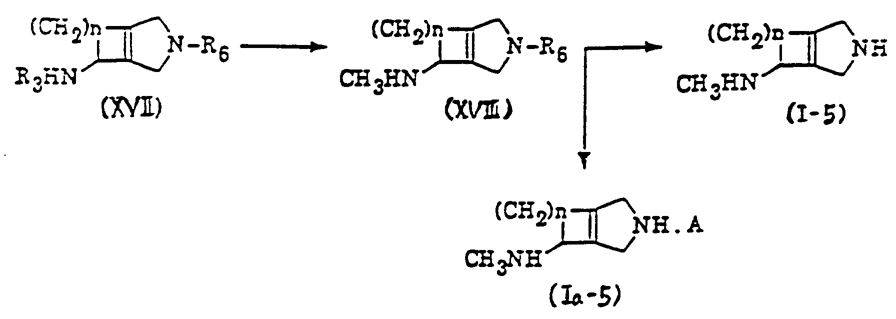 wherein n, A, R₃ and R₆ are as defined above and R₆ is p-toluene sulfonyl or methanesulfonyl.
